# EUROPEAN PATENT APPLICATION

(11) **EP 4 183 779 A1**
(43) Date of publication of application: **24.05.2023**
(21) Application number: 21209082.3
(22) Date of filing: 18.11.2021
(51) Int. Cl.: C07D 307/54

(54) **MALONATE AND FURAN BASED SURFACTANTS**

(71) Applicant: NEDERLANDSE ORGANISATIE VOOR TOEGEPAST- NATUURWETENSCHAPPELIJK ONDERZOEK TNO, 2595 DA 's-Gravenhage (NL)
(72) Inventor: CROCKATT, Marc, 2595 DA 's-Gravenhage (NL)
(74) Representative: V.O.

(57) **Abstract**

The invention is directed to a method for the preparation of a surfactant, said method comprising reacting a furanic compound according to formula (V), or a salt thereof, with a malonate according to formula (VI), or a salt thereof, to obtain a first furan-based surfactant precursor of formula (I) or a salt thereof, 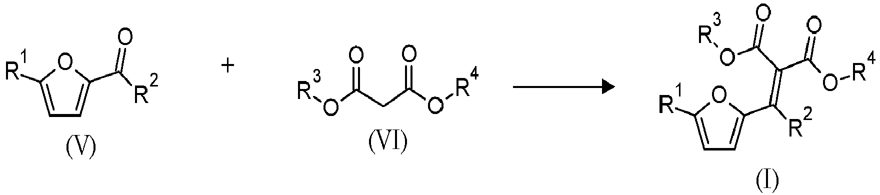 wherein R¹ is H or CH₂OX, wherein X is H or an aliphatic group;
wherein R², R³ and R⁴ are independently H or an aliphatic group;
wherein the method further comprises one or more steps to convert the first furan-based surfactant precursor of formula (I), or a salt thereof, to a surfactant, which surfactant comprises

- a furan-moiety;
- a hydrophilic moiety, preferably selected from the group consisting of sulfate, sulfonate, sulfinate, thiosulfate, sulfamidate, carboxylate, sarcosinate and taurate, phosphate, pyrophosphate, phosphonate, amines or ammonium, polyammonium, hydroxyammonium, pyridinium, picolinium, imidazolinium, benzimidazolinium, oxonium, sulfonium, phosphonium and non-ionic groups, and;
- a hydrophobic moiety comprising an aliphatic chain comprising at least 6 carbon atoms.

## Description

The invention is in the field of surfactants. In particular, the invention is directed to a method to prepare furan-based surfactants from furanic compounds and malonate-based compounds.

Surfactants, or surface-active agents, are chemical compounds that are capable of lowering the surface tension between two liquids or between a gas and a liquid. Surfactants are widely used for a range of applications, such as detergents, emulsifiers, foaming agents and dispersants. Typically, the compounds are amphiphilics, *i.e*. compounds with both a hydrophobic tail and a hydrophilic head. This combination may allow for a water-soluble component and a water-insoluble (*i.e*. oil-soluble) component. Accordingly, the surfactant may absorb at *i.a*. oil/water interphases and can provide interesting properties for a variety of applications.

It is expected that the market for surfactants undergoes continuous growth in the coming years. The main contributor to the market constitutes detergents and cleaners for households as over 50% of all surfactants typically end up in these products. Currently, the largest production comprises petrochemical-based linear alkylbenzene sulfonates (LAS), with around 4 million tons per year being produced and consumed.

LAS surfactants are typically a mixture of compounds based on a hydrophilic sulfonate head attached to a hydrophobic benzene ring which is attached to a hydrophobic alkyl chain. The big advantage of LAS is its tolerance of hard water, which commonly inhibits the functioning of other groups of surfactants. Further, LAS typically has a low critical micelle concentration, low Krafft temperature, fast wetting and good foaming behavior. LAS is accordingly suitable for broad use in a variety of applications.

However, LAS is fossil-based and has a negative environmental impact. Accordingly, there is a strong desire to provide more sustainable surfactants which have comparable or improved properties compared to LAS.

One method to provide surfactants produced from bio-based chemicals such as sugar-derived furans is described in *e.g.* WO2017/079718 and by Park et al. (ACS Cent. Sci. 2016, 2, 11, 820-824). Here, oleo-furan surfactants derived from furan and fatty acids are described. By adjusting the fatty acid the surfactant properties could be altered. Additionally, the surfactants could better tolerate the metal ions contained in hard water, therefore making chelating agents, which are typically added to e.g. detergents, unnecessary. However, the surfactants are synthesized from furan. While it is a bio-based molecule, it requires decarbonylation of furfural to prepare furan, thereby decreasing the atom efficiency and resulting in a waste stream of carbon monoxide. Furthermore, it requires an additional processing step, resulting in increased cost and inefficiency. Moreover, the process may be challenging to scale-up.

Other bio-based surfactants are described by Kipshagen et al. (Green Chem. 2019, 21, 3882). Here *i.a*. furfural and 5-hydroxymethylfurfural (5-HMF) are used as the basis for the surfactants. In the case of furfural, it is converted into tetrahydrofuran followed by side-chain manipulation and the introduction of a hydrophilic head. In the case of HMF, it is first hydrogenated to 2,5-bis(hydroxymethyl)furan (BHMF) followed by side-chain manipulation and introduction of the hydrophilic head.

5-HMF has also been used as a starting material for surfactants as described in WO2016/028845, wherein the final surfactants were prepared by esterification, amination and alkylation to produce non-ionic surfactants. However, these do not mimic LAS.

Other surfactants are described in e.g. WO2021/083642, WO2015/084813 and WO2017/079719.

It is an object of the present inventors to provide an improved method to prepare furan-based surfactants that overcomes at least part of the above-mentioned drawbacks. The present inventors realized that this can be achieved by a process comprising a reaction of a furanic compound and malonic acid or an ester thereof. It was found that this approach advantageously provides access to surfactants with a variety of hydrophobic tails and hydrophilic heads, enabling fine-tuning of the furan-based surfactant specific to the particular application of the surfactant. The method according to the present invention is further advantageously efficient and suitable to be scaled-up.

Figure 1 illustrates a number of reactive sites of a furan-based surfactant precursor according to the present invention.

Figure 2 illustrates a schematic overview of the possible reaction paths according to preferred embodiments of the present invention.

Accordingly, in a first aspect the present invention is directed to a method for the preparation of a surfactant, said method comprising reacting a furanic compound according to formula (V) with a malonate according to formula (VI) to obtain a first furan-based surfactant precursor of formula (I), 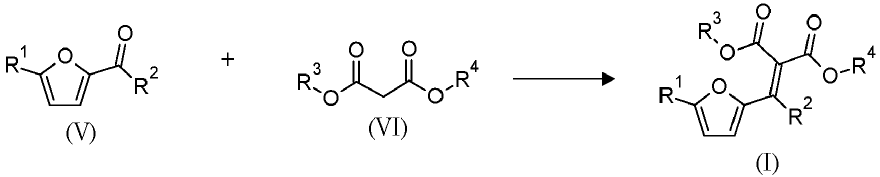
wherein R¹ is H or CH₂OX, wherein X is H or an aliphatic group;
wherein R², R³ and R⁴ are independently H or an aliphatic group.

The method further comprises one or more steps to convert the first furan-based surfactant precursor of formula (I) to a surfactant, which comprises a furan-moiety, a hydrophilic group and a hydrophobic moiety.

The reaction of the furanic compound and the malonate according to the present invention can also generally be referred to as a condensation reaction or a Knoevenagel-type condensation. Suitable methods and reaction conditions for the Knoevenagel-type condensation are disclosed in WO 2018/236218, Van Schijndel et al. Synlett 29 (2018) 15, 1983-1988, and Song et al. Catalysts 6 (2016) 106.

Aliphatic group is herein used to describe aliphatic groups comprising one or more carbon atoms, e.g. methyl, ethyl, propyl and the like. The aliphatic group may be an aliphatic chain. Aliphatic chain is herein used to describe a carbon chain comprising multiple carbon atoms and may be linear, branched, cyclic, saturated and/or unsaturated but not aromatic. Thus, the carbons in the aliphatic chain may be joined by single bonds, one or more carbon pairs may be joined by a double bond and/or one or more carbons may be joined by a triple bond. Also, the chain may comprise one or more non-aromatic carbocyclic moieties. In case the aliphatic chain is branched or cyclic, the main chain (*i.e*. the longest chain of carbon atoms) is considered the backbone chain. The hydrophilic tail may suitably be an aliphatic hydrocarbon chain or an aliphatic ether. Preferably, if the aliphatic chain comprises a certain number of carbon atoms, e.g. at least 6 carbon atoms, it is preferred that the backbone chain comprises said number of carbon atoms, e.g. at least 6 carbon atoms. The branch may on itself also be an aliphatic chain.

The terms polar, apolar, hydrophilic and hydrophobic are commonly used in the field of surfactants. It is commonly known that a surfactant is amphiphilic and comprises hydrophobic (or apolar) and hydrophilic (or polar) moieties. In general, hydrophilic is used to describe the capacity of a molecular entity or of a substituent to interact with polar solvents, in particular with water, or with other polar groups and the tendency to mix, be wetted and/or dissolve in water. Typically, hydrophobic is used for moieties that form Van der Waals bonds and minimal to no hydrogen bonds. For instance, aliphatic chains may be considered hydrophobic, while sulfonic and carboxylic acid groups are considered hydrophilic.

The hydrophilic group that is part of the surfactant in accordance with the present invention is preferably selected from the group consisting of sulfate, sulfonate, sulfinate, thiosulfate, sulfamidate, carboxylate, sarcosinate and taurate, phosphate, pyrophosphate, phosphonate, amines or ammonium, polyammonium, hydroxyammonium, pyridinium, picolinium, imidazolinium, benzimidazolinium, oxonium, sulfonium, phosphonium and non-ionic groups.

The furan compound according to formula (V) is preferably furfural (*i.e*. R¹ and R² are H), HMF (*i.e*. R¹ is CH₂OH and R² is H), or a derivative of these.

In particular embodiments, R² of the furan compound according to formula (V) is an aliphatic group. Such a furan compound can suitably be prepared from a precursor according to formula (V) wherein R² is H, followed by an oxidation, as illustrated in Scheme 1, wherein R¹ is H or CH₂OX, wherein X is H or an aliphatic group and R² is an aliphatic group.

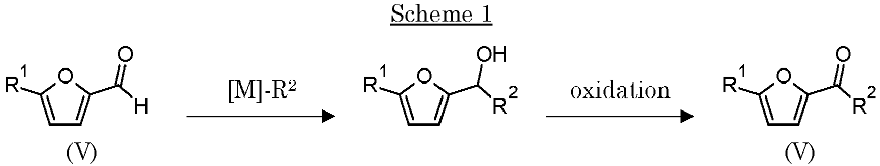

The addition of the aliphatic group R² as illustrated in Scheme 1 can be carried out using well-known chemistry, including for example reaction with an organometallic nucleophile ([M]-R²) in the Grignard reaction or the related Barbier reaction (see *e.g.* Clayden et al. Organic Chemistry, Oxford University Press and WO 2015/084813). The oxidation to obtain (V) in Scheme 1 can for instance be carried out using well-known chemistry (see *i.a*. Clayden et al. Organic Chemistry, Oxford University Press), but also using more specialized catalyzed processes as described in Ye et al., Chem. Commun., 56 (2020) 11811-11814.

In particular embodiments, the furan compound of formula (V) has an R¹ that is CH₂OX wherein X is an aliphatic group. Such a compound can be obtained as a product from a dehydration and hydrolysis process of a sugar, wherein an alcohol is applied in *situ.* Alternatively, such a compound can be obtained by alkylating a precursor according to formula (V) wherein R¹ that is CH₂OH, as illustrated in Scheme 2, with a compound X-Y wherein X is an aliphatic group and Y is a leaving group, *e.g.* a halide such a bromide. Alkylation of alcohols is also well-known chemistry (see *i.a*. Clayden et al. Organic Chemistry, Oxford University Press).

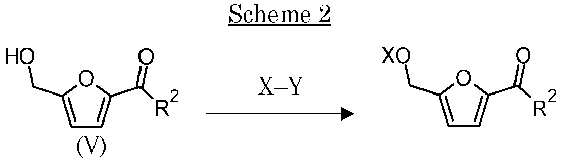

The malonate compound according to formula (VI) can be malonic acid (*i.e*. R³ and R⁴ are H), a monoester (*i.e*. R³ or R⁴ is H and R³ or R⁴ is an aliphatic group) or a diester (*i.e*. R³ and R⁴ are both an aliphatic group, which may be the same of different). Malonic acid and various mono- and diester are commercially available and others can easily be prepared using standard esterification methods known to the skilled person.

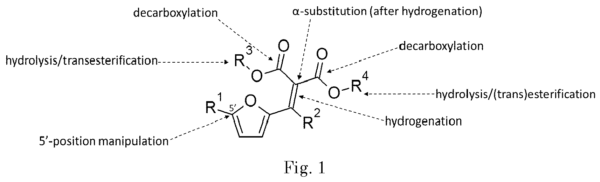

The present inventors found that the first furan-based surfactant precursor of formula (I) is multifunctional, meaning it can be manipulated in a variety of reaction processes to readily access a large variety of surfactant products. As illustrated in Figure 1, these reaction processes include hydrogenation, decarboxylation, 5'-position manipulation, hydrolysis, esterification and/or transesterification, and hydrogenation followed by α-substitution. A combination of these processes, either in one or in subsequent reaction steps is also possible.

The present inventors recognized that the one or more steps to convert the first furan-based surfactant precursor of formula (I) may be one, two or three steps selected from the group consisting of hydrogenation, decarboxylation, 5'-position substitution or manipulation, and α-substitution. More steps are theoretically possible, but realistically not feasible in view of overall process efficiency and/or economics. However, hydrolysis, esterification and/or transesterification of the -CO₂R³ and -CO₂R⁴ are relatively facile steps and can be carried out in addition to said group of reactions.

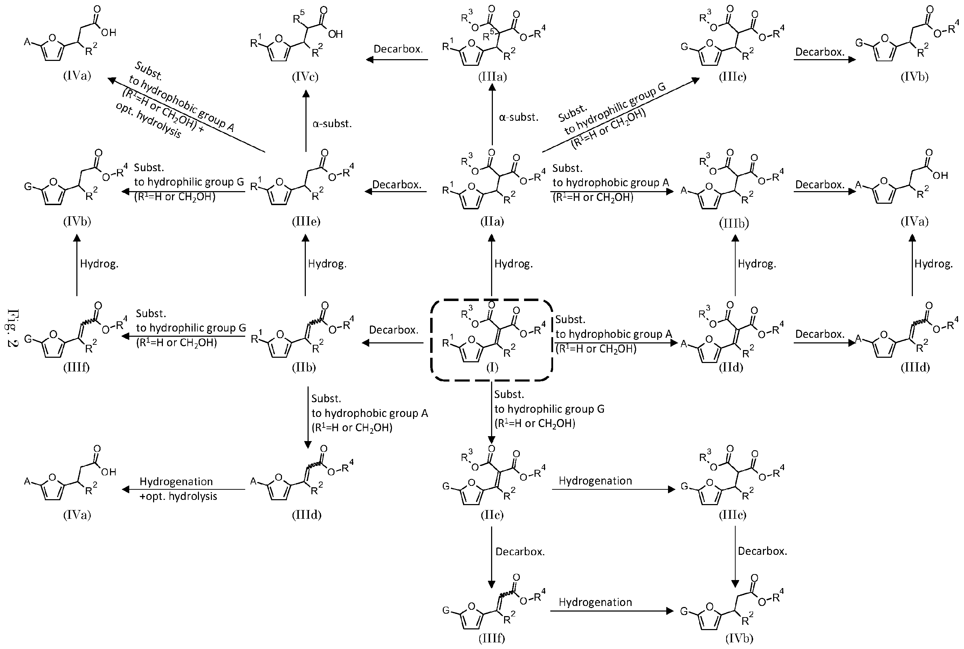

Accordingly, as illustrated in Figure 2, the first furan-based surfactant precursor of formula (I) may be converted in one, two or three step into the compounds included in Table 1. These compounds are referred herein to first, second and third-level compounds, depending on the number steps is required to prepare the compounds from the first furan-based surfactant precursor (excluding hydrolysis, esterification and/or transesterification).

**Table 1 - second, third and fourth level compounds**

| *Second-level compounds:* | |
|---|---|
| 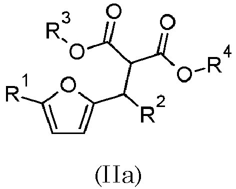 | 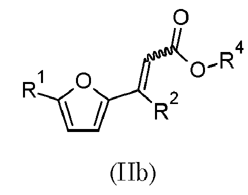 |
| 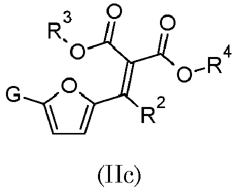 | 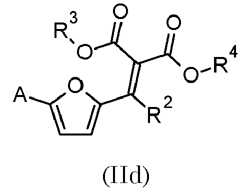 |

| *Third-level compounds:* | |
|---|---|
| 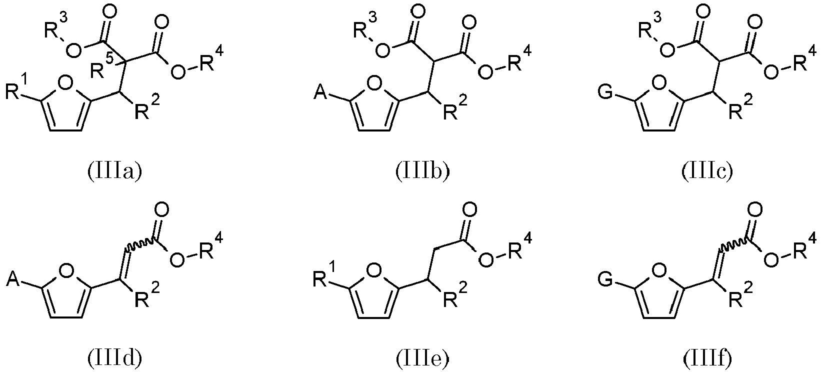 | |

| *Fourth-level compounds:* | |
|---|---|
| 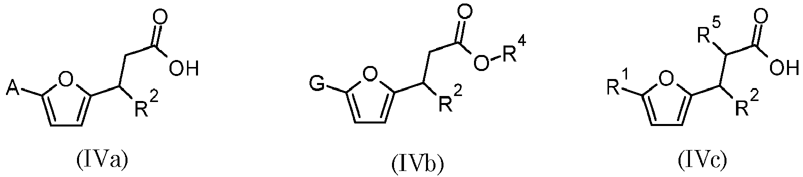 | |

The labels A and G in any of the formulae in Table 1 represent hydrophobic and hydrophilic substituents, respectively. This is irrespective of whether the compound concerned can act as the surfactant or requires one or more further steps before the compound can act as such. This may be differed however for the substituents which are represented by R¹-R⁵. These substituents can suitably be selected based on whether the compound concerned can act as the surfactant, or whether the compound is continued in one or more further steps before the surfactant is formed. Namely, if the compound can act as the surfactant, it comprises both a hydrophilic and a hydrophobic moiety. On the other hand, if the compound is continued in one or more further steps, the substituents which are represented by R¹-R⁵ can suitably be converted or introduced (*e*.*g*. by hydrolysis, esterification, transesterification and the like) to obtain a compound that can act as a surfactant in a later stage. In the embodiments wherein the compound of any of the formulae in Table 1 is the surfactant according to the present invention, the options that R¹-R⁵ can be are thus restricted vis-à-vis the options for formula (I). On the other hand, in the embodiments wherein the compound is continued in one or more steps to obtain the surfactant, R¹-R⁵ can be as follows for any of formulae (IIa)-(IId) and (IIIa)-(IIIf): R¹ is H or CH₂OX, wherein X is H or an aliphatic group; R², R³ and R⁴ are independently H or an aliphatic group; and R⁵ is an aliphatic group.

Thus, in particular embodiments, the surfactant in accordance with the present invention has any of the formulae included in Table 2.

**Table 2 - Surfactants**

| Formula | Substituents |
|---|---|
| 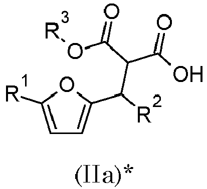 | R¹ is H or CH₂OX, wherein X is H or an aliphatic group, R² and R³ are independently H or an aliphatic group, provided that that at least one of R¹-R³ is an aliphatic chain comprising more than 6 carbon atoms. |
| 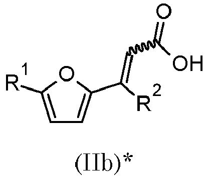 | R¹ is H or CH₂OX, wherein X is H or an aliphatic group, R² is H or an aliphatic group, provided that at least one of R¹ and R² is an aliphatic chain comprising more than 6 carbon atoms. |
| 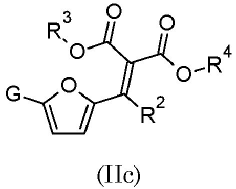 | R², R³ and R⁴ are independently H or an aliphatic group, provided that at least one of R², R³ and R⁴ is an aliphatic chain comprising more than 6 carbon atoms. |
| 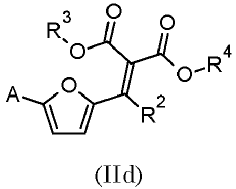 | R², R³ and R⁴ are independently H or an aliphatic group, provided that at least one of R³ and R⁴ is H. |
| 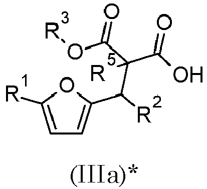 | R¹ is H or CH₂OX, wherein X is H or an aliphatic group, R² and R³ are independently H or an aliphatic group, R⁵ is an aliphatic chain comprising more than 6 carbon atoms. |
| 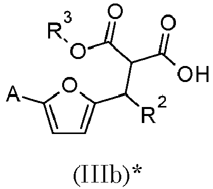 | R² and R³ are independently H or an aliphatic group. |
| 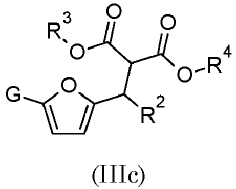 | R², R³ and R⁴ are independently H or an aliphatic group, provided that at least one of R², R³ and R⁴ is an aliphatic chain comprising six or more carbon atoms. |
| 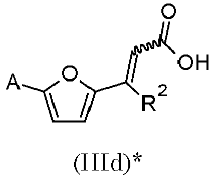 | R² is H or an aliphatic group. |
| 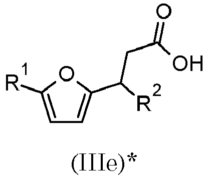 | R¹ is H or CH₂OX, wherein X is H or an aliphatic group, R² is H or an aliphatic group, provided that at least one of R¹ and R² is an aliphatic chain comprising six or more carbon atoms. |
| 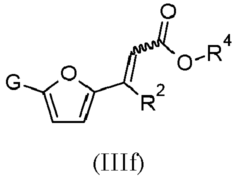 | R² and R⁴ are independently H or an aliphatic group, provided that at least one of R² and R⁴ is an aliphatic chain comprising six or more carbon atoms. |
| 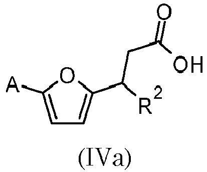 | R² is H or an aliphatic group. |
| 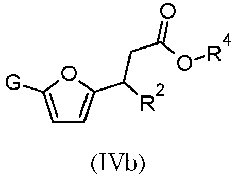 | R² and R⁴ are independently H or an aliphatic group, provided that at least one of R² and R⁴ is an aliphatic chain comprising six or more carbon atoms. |
| 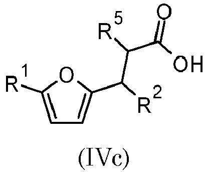 | R¹ is H or CH₂OX, wherein X is H or an aliphatic group, R² is H or an aliphatic group, R⁵ is an aliphatic chain comprising more than 6 carbon atoms. |

| | |
|---|---|
| * R⁴ is H. | |

It may be appreciated that in the embodiments wherein the compound of any of formulae (IIa)-(IId) and (IIIa)-(IIIf) is the surfactant, said compound comprises a hydrophilic moiety represented by G and/or by at least one carboxylate originating from the malonate (*i.e*. R⁴ and/or R³ are/is H), the latter being particularly preferred. The hydrophilic moiety preferably originates from the malonate for sake of atom efficiency. More preferably, the hydrophilic group is a dicarboxylate originating from the malonate (*i.e*. R⁴ and R³ are H), for an even better atom efficiency. Accordingly, the surfactant is preferably of any of formulae (IIa), (IIIa) and (IIIb), with R³ and R⁴ both being H. The formulae (IIa) and (IIIb), with R³ and R⁴ both being H are most preferred in this respect. Accordingly, a method including providing (I), and converting (I) into (IIa) or (IIIb), optionally via (IId) is a particularly preferred embodiment.

For any of formulae (IIa)-(IId), (IIIa)-(IIIf) and (IVa)-(IVc), G represents a hydrophilic moiety. In particular embodiments wherein R¹ = H *(e.g.* if furfural is used as a starting material), G may represent the hydrophilic group. In particular embodiments wherein R¹ = CH₂OH, *(e.g.* if HMF is used as a starting material), G may represent a -CH₂-hydrophilic group.

The hydrophilic moiety may thus be an ionic moiety, e.g. an anionic, a cationic, or a non-ionic moiety. Examples of suitable non-ionic moieties that G may comprise include poly(ethylene oxide), poly(co-ethylene oxide co-propylene oxide) (also referred to as poloxamers), polyglycosides, isosorbide and its derivatives, 1,4-sorbitane and its derivatives, and the like. These moieties can readily be introduced by a reaction of the hydroxyl group of the first furan-based surfactant precursor (also referred to as the first level compound), and of the second and third level compounds wherein R¹ is CH₂OH.

The hydrophilic group is preferably an ionic group and can be present with a counter-ion to balance the charges. It may be appreciated that the counter-ions can be any ion that balances the charge, for instance, if the hydrophilic group has a monovalent negative charge, the counter-ion can be e.g. sodium (Na⁺), potassium (K⁺), lithium (Li⁺) and/or ammonium (NH₄⁺). Preferably, the counter-ion is an ammonium ion, an alkali metal ion or alkaline earth metal ion. Suitable hydrophilic groups and counter-ions are for instance described in WO2017/079719.

The hydrophilic group, and preferably G as such, is preferably selected from the group consisting of sulfate (-O-SO_{3⁻}), sulfonate (-SO_{3⁻}), sulfinate (-SO_{2⁻}), thiosulfate (-O-S₂O_{2⁻}), sulfamidate (-NH-SO_{3⁻}), carboxylate (-CO_{2⁻}), sarcosinate and taurate (-NR-R-CO_{2⁻}), phosphate (-O-PO_{3⁻} or -O-PO₂-OR-), pyrophosphate (-O-PO₂-O-PO₂-OR²⁻), phosphonate (-PO₂R- or -PO_{3⁻}), amines or ammonium (-NR₃⁺), polyammonium (-NR₂-R-NR₃²⁺), hydroxyammonium (-NR₂OH⁺), pyridinium (-NC₅H₅⁺), picolinium (-NC₅H₅-R⁺), imidazolinium 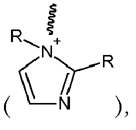 benzimidazolinium ( 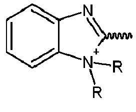 ), oxonium (-OR_{2⁺}), sulfonium (-SR_{2⁺}) and phosphonium (-PR_{3⁺}). Herein 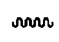 represents the bond to the furanic ring and R is H or a C₁-C₃ alkyl. Particularly good surfactant properties are obtained for sulfonate as hydrophilic group.

In a particular embodiment, the first level compound is such that R¹ is CH₂OH, which can directly or indirectly be manipulated to a hydrophilic group G. In such embodiments, G may be selected from the group consisting of methylene sulfate (-CH₂O-SO_{3⁻}), methylene sulfonate (-CH₂SO_{3⁻}), methylene sulfinate (- CH₂SO_{2⁻}), methylene thiosulfate (-CH₂O-S₂O_{2⁻}), methylene sulfamidate (-CH₂NH- SO_{3⁻}), methylene carboxylate (-CH₂CO_{2⁻}), methylene sarcosinate and taurate (- CH₂NR-R-CO_{2⁻}), methylene phosphate (-CH₂O-PO_{3⁻} or - CH₂O-PO₂-OR-), methylene pyrophosphate (-CH₂O-PO₂-O-PO₂-OR²⁻), methylene phosphonate (-CH₂PO₂R- or - CH₂PO_{3⁻}), methylene amines or ammonium (-CH₂NR₃⁺), methylene polyammonium (-CH₂NR₂-R-NR₃²⁺), methylene hydroxyammonium (-CH₂NR₂OH⁺), methylene pyridinium (-CH₂NC₅H₅⁺), methylene picolinium (-CH₂NC₅H₅-R⁺), methylene imidazolinium 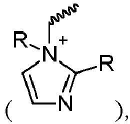 methylene benzimidazolinium 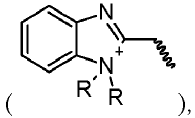 methylene oxonium (-CH₂OR_{2⁺}), methylene sulfonium (-CH₂SR_{2⁺}) and methylene phosphonium (-CH₂PR_{3⁺}), wherein R is H or a C₁-C₃ alkyl, preferably H or Me.

For any of formulae (IIa)-(IId), (IIIa)-(IIIf) and (IVa)-(IVc), A represents a hydrophobic moiety. The hydrophobic moiety comprises an aliphatic chain. Notably, R² and R³ may also comprise an aliphatic chain, even if the compound comprises substituent A, in which embodiments the compound thus comprises more than one aliphatic chain. Unless explicitly specified differently herein, an aliphatic chain preferably comprising at least 4 carbon atoms, more preferably at least 6 carbon atoms, even more preferably between 6 to 26 carbon atoms, most preferably between 6 and 18 carbon atoms. Aliphatic chains comprising at least 6 carbon atoms can suitably be used as hydrophobic tails.

The first level compound of formula (I), and in particular embodiments also the second and/or third level compound of any of formulae (IIa)-(IId) and (IIIa)-(IIIf) is continued on one or more steps to obtain the surfactant. These steps preferably comprise hydrogenation, decarboxylation, 5'-position manipulation, preferably with a reactant comprising a hydrophobic group, hydrogenation followed by α-substitution or a combination thereof. And in addition to these reactions, hydrolysis, esterification and or transesterification may be carried out to manipulate the R³ and R⁴ groups. These steps and conversions are generally well-established and basic principle well-known, see *e.g.* Clayden et al. Organic Chemistry, Oxford University Press.

The **hydrogenation** can be carried out with the compound of any of formulae (I), (IIa)-(IId) and (IIIa)-(IIIf) that comprises a C-C double bond, *i.e*. (I), (IIb), (IIc), (IId), (IIId) and (IIIf), leading to a second, third or fourth level compound of formula (IIa), (IIIe), (IIIc), (IIId), (IVa) or (IVb), respectively. Suitable methods and reaction conditions are for instance those disclosed in Coutant et al Beilstein J. Org. Chem. 14 (2018) 2853-2860.

The **hydrogenation** can be optionally followed by **an α-substitution,** which can be carried out with the compound of any of formulae (I), (IIa)-(IId) and (IIIa)-(IIIf) of which the C-C double bond is reduced, but it preferably carried out with the compound of any of formulae (IIa) and (IIIe), leading to a third or fourth level compound of formula (IIIa) or (IVc), respectively. Suitable methods and reaction conditions for the α-substitution are for instance those disclosed in Arai et al. Tetrahedron Letters 51 (2010) 1273-1275 and Schelkun et al. Bioorg. Med. Chem. Lett. 16 (2006) 2329-2332.

The **decarboxylation** can be carried out with the compound of any of formulae (I), (IIa)-(IId) and (IIIa)-(IIIf) that comprises a β-diester moiety, *i.e*. (I), (IIa), (IIc), (IId), (IIIa), (IIIb) and (IIIc), leading to a second, third or fourth level compound of formula (IIb), (IIIe), (IIIf), (IIId), (IVc), (IVa) and (IVb), respectively. Suitable methods and reaction conditions are for instance those disclosed in Mohite and Bhat Org. Lett. 15 (2013) 17, 4564-4567 and Schuppan Chem. Commun. (2004) 792-793.

The **5'-position manipulation** can be carried out with the compound of any of formulae (I), (IIa)-(IId) and (IIIa)-(IIIf) that comprises a nucleophilic site on the 5'-position of the furan, *i.e*. (I), (IIa), (IIb), (IIIa) and (IIIe), provided that R¹ of these compounds is H or CH₂OH.For embodiments wherein said compound has an R¹ that is H, the furan ring is relatively activated and nucleophilic, which allows a nucleophilic substitution of the 5'-position of the furan ring. For embodiments wherein said compound has an R¹ that is CH₂OH, the alcohol group is relatively activated and nucleophilic, which allows a manipulation of the 5'-position of the furan ring. In both of these types of embodiments, introduction of a hydrophobic (A) or a hydrophilic moiety (G) is possible. Suitable methods and reaction conditions for the instruction of a hydrophobic moiety such as an aliphatic group is are for instance those disclosed in Asta et al. Green Chem. 13 (2011) 3066-3069.

The skilled person can suitably select appropriate reactions to introduce the hydrophilic group to the appropriate first, second or third level compound of any of formulae (I), (IIa), (IIb), (IIIa) and (IIIe), provided that R¹ of these compounds is H or CH₂OH.For example, carboxylate or sulfonate as the hydrophilic group may be introduced by reacting the appropriate first, second or third level compound with carbon dioxide or sulfur trioxide, respectively (see also Sung Park et al. ACS Cent. Sci. 2 (2016) 11, 820-824. For the embodiments wherein R¹ is CH₂OH, the hydroxyl can act as a nucleophile to suitably introduce the hydrophilic group.

The hydrolysis, esterification and/or transesterification can be carried out with the compound of any of formulae (I), (IIa)-(IId) and (IIIa)-(IIIf) in any stage of the method. Accordingly, it may be appreciated if the first level compound is converted into a second level compound, which is then optionally further converted into a third level compound, which is in turn then also optionally further converted into a fourth level compound, the R³ and/or R⁴ group of the subsequent first, second, third and fourth level compound do not necessarily have to be the same. For example, in a particular embodiment, the first level compound may be according to formula (I) wherein R³ and R⁴ are both H, and R³ and R⁴ are converted in a aliphatic group (*i.e*. esterification) in a further stage of the overall method to prepare the surfactant. And vice versa, in another particular embodiment, the first level compound may be according to formula (I) wherein R³ and R⁴ are both Me, and R³ and R⁴ are converted in H (*i.e*. hydrolysis) in a further stage of the overall method to prepare the surfactant. In yet another exemplary embodiment, the first level compound may be according to formula (I) wherein R³ and R⁴ are both Me, and R³ and R⁴ are converted in an aliphatic chain (*i.e*. transesterification) in a further stage of the overall method to prepare the surfactant. Esterification, transesterification and hydrolysis can be carried out in *situ* with the other one or more step described herein to prepare the surfactant. For example, the decarboxylation of β-diester moiety to obtain a mono-ester a may comprise hydrolysis as well. Suitable conditions for the hydrolysis and (trans)esterification are disclosed in WO 2018/236218.

Figure 2 illustrates an overview of the possible reaction pathways that can be taken to convert the first furan-based surfactant precursor of formula (I). It may however be appreciated that any of the routes and formulae are individually according to the present invention. Also, a plurality of routes to a particular compound is according to the present invention. For the purpose of clarity and a concise description, features, routes, compounds and formulae are described herein as part of the same or separate embodiments, however, it will be appreciated that the scope of the invention may include embodiments having combinations of all or some of the features described.

It may be appreciated that any of the compounds, including the first, second, third and fourth level compounds, as well as the surfactant described herein may be in a salt form. In particular the compounds comprising an ionic group (*e*.*g*. carboxylate, sulfonate, etc.) may be in a salt form with a counter ion.

Further, it may be appreciated that any of the compounds may be a single stereoisomer, a mixture of stereoisomers, a single regio-isomer or a mixture of regio-isomers, whenever applicable. Thus, if not explicitly indicated differently, each formula herein represents an enantiomerically pure, a mixture of enantiomers *(e.g.* a racemic mixture), a mixture of diastereoisomers, a single regio-isomer and/or a mixture of regio-isomers, whenever and whatever applicable. A wiggle bond attached to a double bond indicates specifically that the configuration of the substituents on the double bond is undefined and the compound may thus be cis (Z), *trans* (E), or a mixture thereof.

As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features.

The invention may further be illustrated by the following non-limiting examples.

### Example 1 - Synthesis of 2-(2-furylmethylene)malonic acid

A reactor was charged with furfural (14.4 g) and malonic acid (15.02 g). The mixture was heated to 50 °C with stirring, then ammonium bicarbonate (1.19 g) was added. The reaction mixture was stirred at 50 °C for 1 hour then cooled to ambient temperature. Saturated sodium bicarbonate solution (100 mL) was charged, then the stirred mixture was acidified to pH 2 with 1 M aqueous hydrochloric acid to induce precipitation. The mixture was cooled to ~5 °C and the formed solid was isolated by filtration, washing with minimal ice cold water. The resulting solid was dried in a vacuum oven (60 °C) to yield 12.8 g, 50%. Analysis by NMR confirmed the product to be 2-(2-furylmethylene)malonic acid.

### Example 2 - Synthesis of dimethyl 2-(2-furylmethylene)malonate

A reactor was charged with furfural (14.4 g) and dimethyl malonate (24 g). The mixture was heated to 90 °C with stirring, then ammonium bicarbonate (1.19 g) was added. The reaction mixture was stirred at 90 °C for 3 hours then cooled to ambient temperature. The mixture was diluted with ethyl acetate (20 mL) and the organics washed with brine (20 mL). The organics were dried (Na₂SO₄), filtered and concentrated to an oil, which was purified by flash chromatography. Concentration of the appropriate fractions resulted in the precipitation of a solid which was dried in a vacuum oven (60 °C) to yield 19.5 g, 56%. Analysis by NMR confirmed the product to be dimethyl 2-(2-furylmethylene)malonate.

## Claims

1. Method for the preparation of a surfactant, said method comprising reacting a furanic compound according to formula (V), or a salt thereof, with a malonate according to formula (VI), or a salt thereof, to obtain a first furan-based surfactant precursor of formula (I) or a salt thereof, 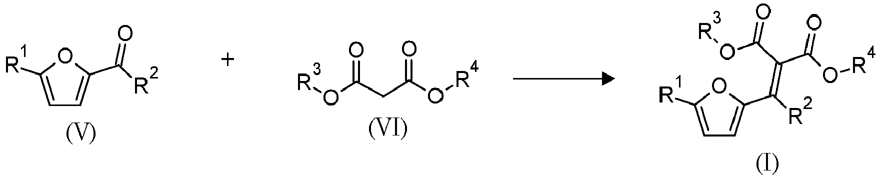
wherein R¹ is H or CH₂OX, wherein X is H or an aliphatic group;
wherein R², R³ and R⁴ are independently H or an aliphatic group;
wherein the method further comprises one or more steps to convert the first furan-based surfactant precursor of formula (I), or a salt thereof, to a surfactant, which surfactant comprises
- a furan moiety;
- a hydrophilic moiety, preferably selected from the group consisting of sulfate, sulfonate, sulfinate, thiosulfate, sulfamidate, carboxylate, sarcosinate and taurate, phosphate, pyrophosphate, phosphonate, amines or ammonium, polyammonium, hydroxyammonium, pyridinium, picolinium, imidazolinium, benzimidazolinium, oxonium, sulfonium, phosphonium and non-ionic groups, and;
- a hydrophobic moiety comprising an aliphatic chain comprising at least 6 carbon atoms.

2. Method according to the previous claim, wherein the one or more steps to convert the first furan-based surfactant precursor of formula (I), or a salt thereof, to a surfactant comprise a hydrogenation, decarboxylation, 5'-position manipulation, preferably with a reactant comprising a hydrophobic group, hydrogenation followed by α-substitution or a combination thereof.

3. Method according to any of claims 1-2, wherein the one or more steps to convert the first furan-based surfactant precursor of formula (I), or a salt thereof, to a surfactant comprise reacting the first furan-based surfactant precursor of formula (I), or a salt thereof, to obtain a second-level compound of formula (IIa), or a salt thereof, comprising hydrogenation; 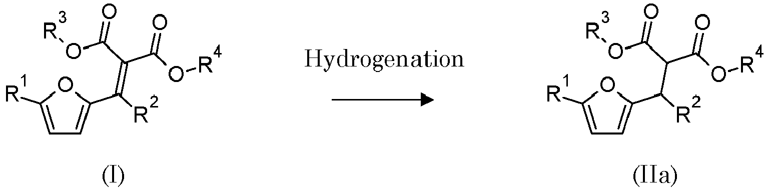
wherein for formula (I): R¹, R², R³ and R⁴ are as defined for formula (I) in claim 1; and
wherein the second-level compound of formula (IIa), or a salt thereof, is continued in one or more steps to convert the second-level compound of formula (IIa), or a salt thereof, to said surfactant, in which case for formula (IIa): R¹, R², R³ and R⁴ are as defined for formula (I) in claim 1; or
wherein the second-level compound of formula (IIa), or a salt thereof, is said surfactant in which case for formula (IIa), or a salt thereof, R¹, R², R³ and R⁴ are as defined for formula (I) in claim 1, provided that that at least one of R¹-R³ is an aliphatic chain comprising more than 6 carbon atoms and R⁴ is H, preferably R³ and R⁴ are H.

4. Method according to any of claims 1-2, wherein the one or more steps to convert the first furan-based surfactant precursor of formula (I), or a salt thereof, to a surfactant comprise reacting the first furan-based surfactant precursor of formula (I), or a salt thereof, to obtain a second-level compound of formula (IIb), or a salt thereof, comprising decarboxylation; 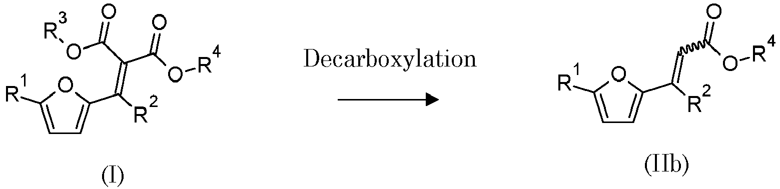
wherein for formula (I): R¹, R², R³ and R⁴ are as defined for formula (I) in claim 1; and
wherein the second-level compound of formula (IIb), or a salt thereof, is continued in one or more steps to convert the second-level compound of formula (IIb), or a salt thereof, to said surfactant, in which case for formula (IIb): R¹, R² and R⁴ are as defined for formula (I) in claim 1; or
wherein the second-level compound of formula (IIb), or a salt thereof, is said surfactant in which case for formula (IIb) R¹ and R² are as defined for formula (I) in claim 1 provided that at least one of R¹ and R² is an aliphatic chain comprising more than 6 carbon atoms and R⁴ is H.

5. Method according to any of claims 1-2, wherein the one or more steps to convert the first furan-based surfactant precursor of formula (I), or a salt thereof, to a surfactant comprise reacting the first furan-based surfactant precursor of formula (I), or a salt thereof, wherein R¹ is H or CH₂OH, to obtain a second-level compound of formula (IIc), or a salt thereof, comprising a substitution on the furan with a reactant to introduce hydrophilic group G; 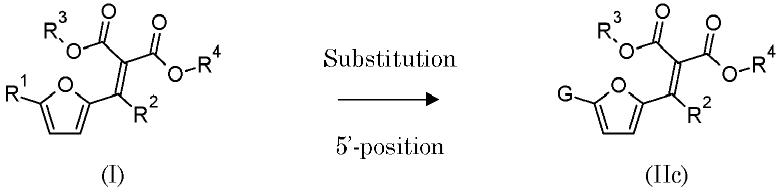 wherein G is selected from the group consisting of sulfate, sulfonate, sulfinate, thiosulfate, sulfamidate, carboxylate, sarcosinate and taurate, phosphate, pyrophosphate, phosphonate, amines or ammonium, polyammonium, hydroxyammonium, pyridinium, picolinium, imidazolinium, benzimidazolinium, oxonium, sulfonium, phosphonium, methylene sulfate, methylene sulfonate, methylene sulfinate, methylene thiosulfate, methylene sulfamidate, methylene carboxylate, methylene sarcosinate and taurate, methylene phosphate, methylene pyrophosphate, methylene phosphonate, methylene amines or ammonium, methylene polyammonium, methylene hydroxyammonium, methylene pyridinium, methylene picolinium, methylene imidazolinium, methylene benzimidazolinium, methylene oxonium, methylene sulfonium, and methylene phosphonium, poly(ethylene oxide), poly(co-ethylene oxide co-propylene oxide) (also referred to as poloxamers), polyglycosides, isosorbide and its derivatives, 1,4-sorbitane and its derivatives, and the like, preferably -SO₃H or -CO₂H;
wherein for formula (I): R², R³ and R⁴ are as defined for formula (I) in claim 1; and wherein the second-level compound of formula (IIc), or a salt thereof, is continued in one or more steps to convert the second-level compound of formula (IIc), or a salt thereof, to said surfactant in which case for formula (IIc): R², R³ and R⁴ are as defined for formula (I) in claim 1; or
wherein the second-level compound of formula (IIc), or a salt thereof, is said surfactant in which case for formula (IIc) R², R³ and R⁴ are as defined for formula (I) in claim 1 provided that at least one of R², R³ and R⁴ is an aliphatic chain comprising more than 6 carbon atoms.

6. Method according to any of claims 1-2, wherein the one or more steps to convert the first furan-based surfactant precursor of formula (I), or a salt thereof, to a surfactant comprise reacting the first furan-based surfactant precursor of formula (I), or a salt thereof, wherein R¹ is H to obtain a second-level compound of formula (IId), or a salt thereof, comprising substitution on the furan with a reactant to introduce hydrophobic group A; 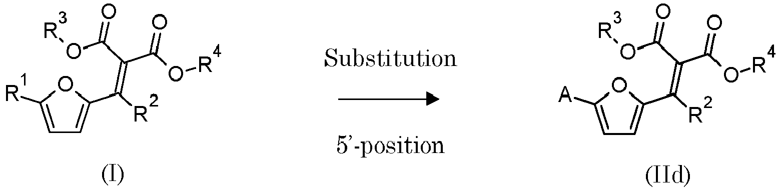 wherein A is an aliphatic chain comprising at least six carbon atoms,
wherein for formula (I): R², R³ and R⁴ are as defined for formula (I) in claim 1; and wherein the second-level compound of formula (IId), or a salt thereof, is continued in one or more steps to convert the second-level compound of formula (IId), or a salt thereof, to said surfactant in which case for formula (IId): R², R³ and R⁴ are as defined for formula (I) in claim 1; or
wherein the second-level compound of formula (IId), or a salt thereof, is said surfactant in which case for formula (IId) R², R³ and R⁴ are as defined for formula (I) in claim 1 provided that at least one of R³ and R⁴ is H, preferably R³ and R⁴ are H.

7. Method according to claim 3 or 4, wherein the one or more steps to convert the second-level compound, or a salt thereof, of formula (IIa) or (IIb) to a surfactant comprise reacting the second-level compound, or a salt thereof, of formula (IIa) or (IIb) wherein R¹ is H or CH₂OH in a reaction comprising a substitution on the furan with a reactant to introduce a hydrophobic group A or a hydrophilic group G, to obtain a third-level compound, or a salt thereof, of any of formulae (IIIb), (IIIc), (IIId) and (IIIf) 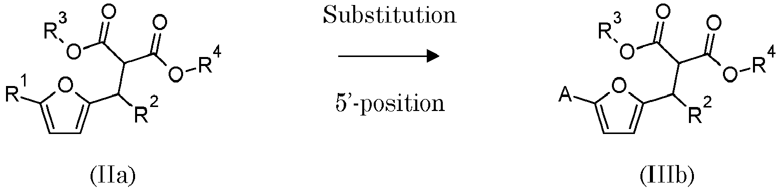 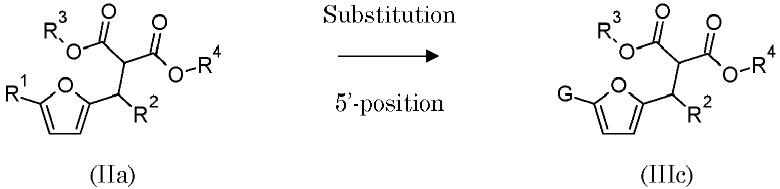 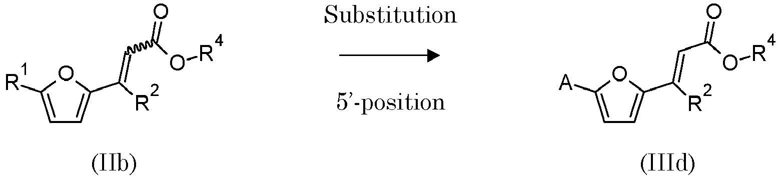 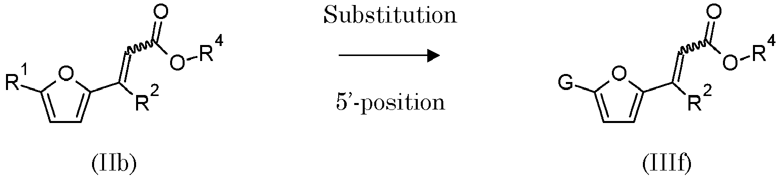 wherein A and G are as defined in claims 5 and 6,
wherein formulae (IIa) and (IIb) are as defined in claims 3 and 4; and
wherein the third-level compound, or a salt thereof, of any of formulae (IIIb), (IIIc), (IIId) and (IIIf) is continued in one or more steps to convert said third-level compound, or a salt thereof, to said surfactant in which case for formulae (IIIb), (IIIc), (IIId) and (IIIf): R², R³ and R⁴ are as defined for formula (I) in claim 1; or wherein the third-level compound, or a salt thereof, of any of formulae (IIIb), (IIIc), (IIId) and (IIIf) is said surfactant in which case
for formula (IIIb): R², R³ and R⁴ are as defined for formula (I) in claim 1, provided that least R⁴ is H, preferably R³ and R⁴ are H;
for formula (IIIc): R², R³ and R⁴ are as defined for formula (I) in claim 1, provided that at least one of R², R³ and R⁴ is an aliphatic chain comprising six or more carbon atoms;
for formula (IIId): R² is as defined for formula (I) in claim 1, and R⁴ is H;
for formula (IIIf) R² and R⁴ are as defined for formula (I) in claim 1, provided that at least one of R² and R⁴ is an aliphatic chain comprising six or more carbon atoms.

8. Method according to claim 3, wherein the one or more steps to convert the second-level compound, or a salt thereof, of formula (IIa) to a surfactant comprise reacting the second-level compound, or a salt thereof, of formula (IIa) in a reaction comprising an α-substitution with a reaction comprising R⁵ to obtain a third-level compound, or a salt thereof, of formula (IIIa), optionally followed by reacting the third-level compound, or a salt thereof, of formula (IIIa) in a decarboxylation reaction to obtain the surfactant of formula (IVc), 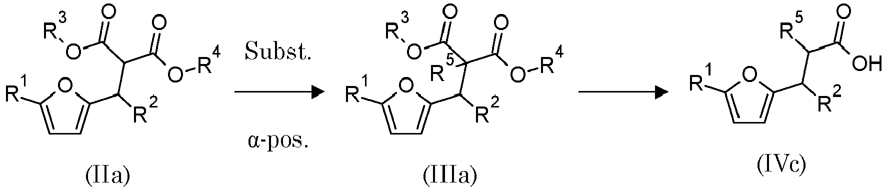 wherein R⁵ is an aliphatic chain comprising at least six carbon atoms;
wherein for formula (IIa) and (IVc): R¹, R², R³ and R⁴ are as defined for formula (I) in claim 1; and
wherein for formula (IIIa) R¹, R², R³ and R⁴ are as defined for formula (I) in claim 1 if the method comprises said optional decarboxylation reaction; or
wherein the third-level compound, or a salt thereof, of formula (IIIa) is said surfactant in which case for formula (IIIa): R¹, R², R³ and R⁴ are as defined for formula (I) in claim 1, provided that at least one of R¹, R², R³ and R⁵ is an aliphatic chain comprising six or more carbon atoms and R⁴ is H, preferably R³ and R⁴ are H.

9. Method according to any of claims 4-6, said method comprising continuing the second-level compound, or a salt thereof, of formula (IIb), (IIc) or (IId) in one or more steps to convert said second-level compound, or a salt thereof, to the surfactant, wherein said one or more steps comprise reacting said second-level compound, or a salt thereof, in a reaction comprising a hydrogenation to obtain a third-level compound, or a salt thereof, of any of formulae (IIIe), (IIIc) and (IIIb) 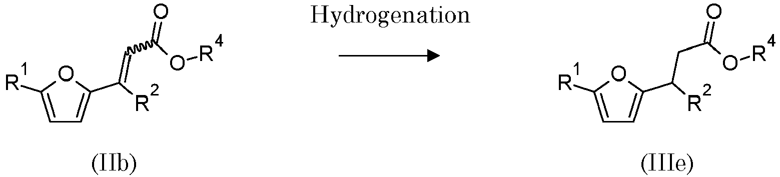 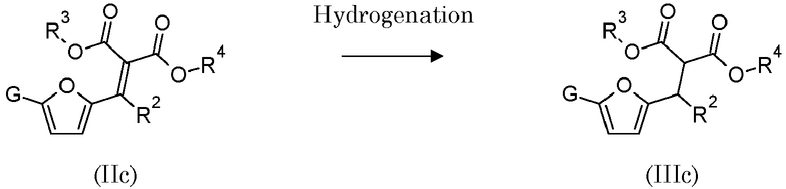 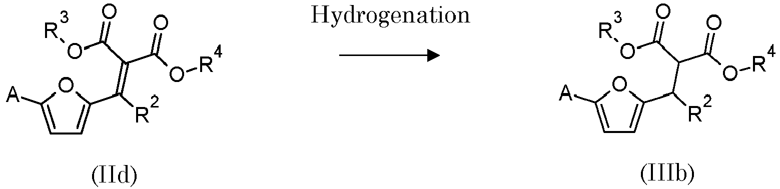 wherein A and G are as defined in claims 5 and 6,
wherein formulae (IIb), (IIc) and (IId) are further as defined in claims 4-6, wherein the third-level compound, or a salt thereof, of any of formulae (IIIe), (IIIc), and (IIIb) is continued in one or more steps to convert said third-level compound, or a salt thereof, to said surfactant in which case for formula (IIIe), (IIIc), and (IIIb):
R¹, R², R³ and R⁴ are as defined for formula (I) in claim 1; or
wherein the third-level compound, or a salt thereof, of any of formulae (IIIe), (IIIc),
and (IIIb) is said surfactant in which case
for formula (IIIe): R¹, R²and R⁴ are as defined for formula (I) in claim 1, provided that at least one of R¹ and R² is an aliphatic chain comprising six or more carbon atoms and R⁴ is H;
for formula (IIIc): R², R³ and R⁴ are as defined for formula (I) in claim 1, provided that at least one of R², R³ and R⁴ is an aliphatic chain comprising six or more carbon atoms; and
for formula (IIIb): R², R³ and R⁴ are as defined for formula (I) in claim 1, provided that R⁴ is H, preferably R³ and R⁴ are H.

10. Method according to claim 3, 5 or 6, said method comprising continuing the second-level compound, or a salt thereof, of formula (IIa), (IIc) or (IId) in one or more steps to convert said second-level compound, or a salt thereof, to the surfactant, wherein said one or more steps comprise reacting said second-level compound, or a salt thereof, in a reaction comprising a decarboxylation to obtain a third-level compound, or a salt thereof, of any of formulae (IIIe), (IIIf) and (IIId) 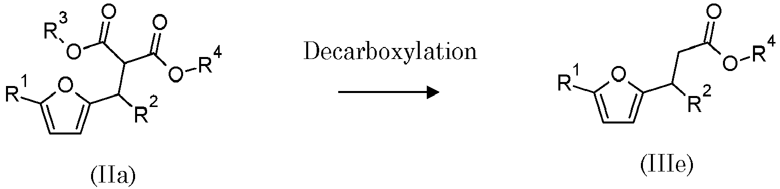 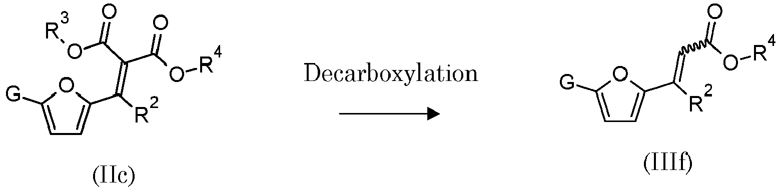 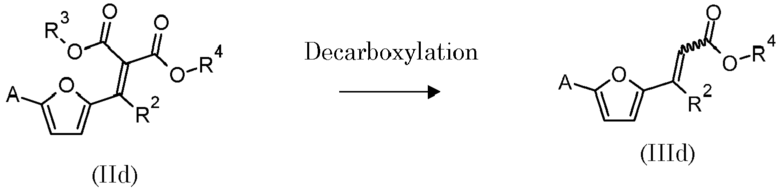 wherein A and G are as defined in claims 5 and 6,
wherein formulae (IIa), (IIc) and (IId) are further as defined in claims 3, 5 and 6, wherein the third-level compound, or a salt thereof, of any of formulae (IIIe), (IIIf) and (IIId) is continued in one or more steps to convert said third-level compound, or a salt thereof, formula to said surfactant in which case for formula (IIIe), (IIIf) and (IIId): R¹, R² and R⁴ are as defined for formula (I) in claim 1; or
wherein the third-level compound, or a salt thereof, of any of formulae (IIIe), (IIIf) and (IIId) is said surfactant in which case
for formula (IIIe): R¹, R² and R⁴ are as defined for formula (I) in claim 1, provided that at least one of R¹ and R² is an aliphatic chain comprising six or more carbon atoms and R⁴ is H;
for formula (IIIh): R² and R⁴ are as defined for formula (I) in claim 1, provided that at least one of R¹ and R² is an aliphatic chain comprising six or more carbon atoms; and
for formula (IIId): R² is as defined for formula (I) in claim 1, and R⁴ is H.

11. Method according to claims 9 or 10, said method comprising continuing the third-level compound, or a salt thereof, of formula (IIIe) in one or more steps to convert said third-level compound, or a salt thereof, to the surfactant, wherein said one or more steps comprise reacting said third-level compound, or a salt thereof, in a reaction comprising a substitution on the furan with a reactant comprising a hydrophobic group A, a substitution on the furan with a reactant comprising a hydrophobic group G or an α-substitution with a reaction comprising R⁵ to obtain the surfactant of formula (IVa) or (IVc), respectively 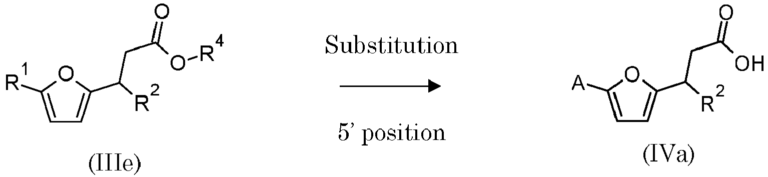 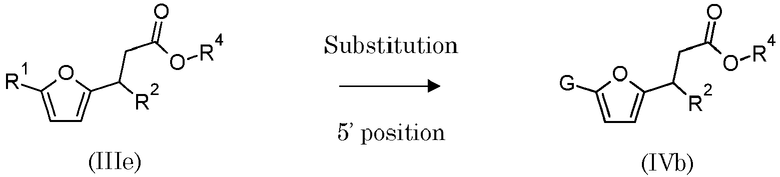 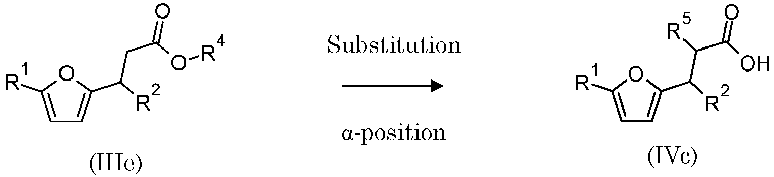 wherein formula (IIIe) is as defined in claims 9 and 10,
wherein for formula (IVa): A is a defined in claim 6 and R² is as defined for formula (I) in claim 1,
wherein for formula (IVb): G as defined in claim 5, preferably -SO₃H or -CO₂H, and R² and R⁴ are as defined for formula (I) in claim 1 provided that at least one of R² and R⁴ is an aliphatic chain comprising more than 6 carbon atoms, and
wherein for formula (IVc): R⁵ is an aliphatic group and R¹ and R² are as defined for formula (I) in claim 1.

12. Method according to any of claim 7, 9 and 10, said method comprising continuing the third-level compound, or a salt thereof, of any of formulae (IIIb), (IIIc), (IIId) and (IIIf) in one or more steps to convert said third-level compound, or a salt thereof, to the surfactant, wherein said one or more steps comprise reacting said third-level compound, or a salt thereof, in a reaction comprising a decarboxylation or in a hydrogenation to obtain the surfactant of any of formulae (IVa) and (IVb) 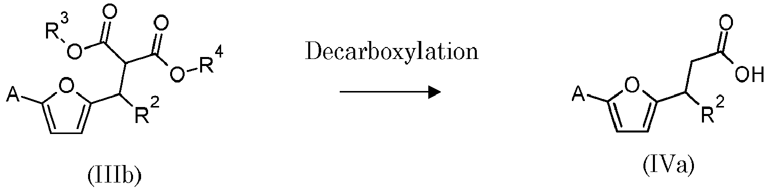 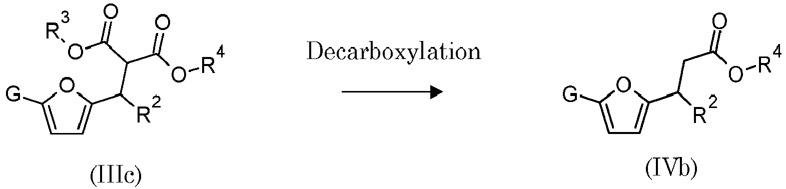 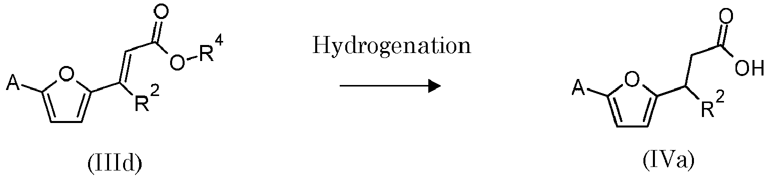 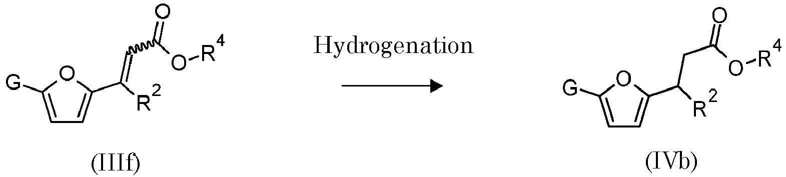
wherein A and G are as defined in claims 5 and 6,
wherein formulae (IIIb), (IIIc), (IIId) and (IIIf) are further as defined in claim 7 or 9, and
wherein for formula (IVa) R² is as defined for formula (I) in claim 1; and
wherein for formula (IVb) R² and R⁴ are as defined for formula (I) in claim 1 provided that at least one of R² and R⁴ is an aliphatic chain comprising more than 6 carbon atoms.

13. Method according to any of the previous claims, wherein for all formulae R¹ is H, preferably wherein for all formulae R¹ and R² are H.

14. Method according to any of the previous claims, wherein for all formulae wherein R³ and R⁴ are H, and preferably wherein R¹ is CH₂OX wherein X is an aliphatic chain.

15. Method according to any of the previous claims, wherein for all formulae R² is an aliphatic chain.
